# EUROPÄISCHE PATENTSCHRIFT

(11) **EP 1 613 149 B1**
(45) Veröffentlichungstag und Bekanntmachung des Hinweises auf die Patenterteilung: **09.03.2011**
(21) Anmeldenummer: 04726868.5
(22) Anmeldetag: 11.04.2004
(51) Int. Cl.: A01M 7/00

(54) **VERFAHREN ZUR KONTROLLE VON SCHÄDLICHEN MIKRO-ORGANISMEN UND INSEKTEN IM PFLANZENSCHUTZ MITTELS DIPOL-ELEKTRISCHER AIR -JET SPRAY-TECHNIK, OZON HALTIGEM WASSER UND UV-C-BESTRAHLUNG**
METHOD FOR CONTROLLING HARMFUL MICRO-ORGANISMS AND INSECTS WITHIN THE CONTEXT OF PLANT PROTECTION BY MEANS OF DIPOLE ELECTRIC AIR JET SPRAY TECHNOLOGY, WATER CONTAINING OZONE AND UV-C RADIATION
PROCEDE POUR CONTROLER DES MICRO-ORGANISMES ET INSECTES NUISIBLES DANS LE CADRE DE LA PROTECTION VEGETALE, AU MOYEN D'UNE TECHNIQUE DE PULVERISATION ELECTRIQUE DIPOLAIRE DE JET D'AIR, GRACE A DE L'EAU CONTENANT DE L'OZONE ET A UN RAYONNEMENT UV-C

(30) Priorität: 11.04.2003 CH 658032003
(43) Veröffentlichungstag der Anmeldung: 11.01.2006
(73) Patentinhaber: Steffen, Hanspeter, 3427 Utzenstorf (CH)
(72) Erfinder: Steffen, Hanspeter, 3427 Utzenstorf (CH)
(74) Vertreter: Roshardt, Werner Alfred
(86) Internationale Anmeldenummer: PCT/CH2004/000254
(87) Internationale Veröffentlichungsnummer: WO 2004/089075

(56) Entgegenhaltungen:
- EP-A- 0 007 459
- EP-A- 1 080 734
- WO-A-01/52626
- PATENT ABSTRACTS OF JAPAN Bd. 0155, Nr. 00 (C-0895), 18. Dezember 1991 (1991-12-18) & JP 3 219808 A (MITSUBISHI HEAVY IND LTD), 27. September 1991 (1991-09-27)
- PATENT ABSTRACTS OF JAPAN Bd. 1998, Nr. 13, 30. November 1998 (1998-11-30) & JP 10 225507 A (NIKKO SUISAN:KK), 25. August 1998 (1998-08-25)

## Beschreibung

### TECHNISCHES GEBIET

Die Erfindung betrifft ein neues Pflanzenschutz - Verfahren mit Ozon und UV-C - Licht und die angewandte Technik zur Durchführung des Verfahrens gemäss dem Oberbegriff der unabhängigen Patentansprüche.

### STAND DER TECHNIK

Zur Bekämpfung von schädlichen Insekten, Pilzen, Bakterien, Viren und Hefen und anderen Schädlingen wurden bis heute im Pflanzenschutz hochgiftige Chemikalien und Substanzen eingesetzt, die auf Nutzpflanzen giftige Rückstände bilden, Resistenzen bei Schädlingen verursachen und die Umwelt schwer belasten und zudem sehr teuer sind.

Der Einsatz von giftigen Chemikalien im Pflanzenschutz ist daher heute sehr umstritten und Konsumenten bevorzugen preiswerte, biologisch und ökologisch Umwelt freundlich hergestellte pflanzliche Nahrungsmittel ohne giftige Inhaltstoffe oder Rückstände.

Mit der neuen Erfindung in der Anwendung von ozonhaltigem Wasser und UV-C-Licht und mit neuer Spray Technik können alle Arten von Pftanzenschädlingen kontrolliert werden, ohne dass giftige und Umwelt schädigende und Resistenzen bildende Substanzen mit Residualwirkung eingestzt werden müssen.

Die neue Pflanzenschutztechnik ist sauber, bedeutend billiger, gleichwertig effizient und vor allem Umwelt freundlich und kann auch im ökologischen und Bio-Anbau eingesetzt werden.

### DARSTELLUNG DER ERFINDUNG

Aufgabe der Erfindung ist die Angabe eines neuen kostengünstigen, Umwelt freundlichen und biologischen Verfahrens zum Schutz von Pflanzen gegen schädliche Insekten-Bakterien-, Viren- und Hefen - Befall ohne Rückstände und Resistenzen bildende Chemikalien unter Einsatz von Ozon haltigem Wasser, Dipol elektrischem Air Jet Spray - Verfahren mit Netzmittel und UV - Licht.

### EINFÜHRUNG

Ozon, 03, ist die 3-atomige Form von Sauerstoff.

Ozon ist hoch reaktiv und das stärkste bekannte Oxidationsmittel. Es ist 51 mal reaktiver als Chlorin und 3125 mal schneller in der Vernichtung von Mikroorganismen.

Ozon ist ein hoch unstabiles Molekül in Gasform, das in kurzer Zeit (ca. 20 Minuten) wieder in Sauerstoff O2 zerfällt.

Ozon wird durch UV-C-Strahlung oder durch Corona Kathoden - Entladung mit ca. 7000 Volt erzeugt.

Ozon ist besser in Wasser löslich als Sauerstoff.

Ozon ist gasförmig, von bläulicher Farbe, stark riechend und bei 0.1 mg pro Liter Luft in permanenter Exposition unschädlich. Ozon wirkt durch das Abspalten eines Sauerstoff - Atoms bei Kontakt mit organischen oder anorganischen Verbindungen und führt zu einer Oxidation, respektive Auflösung von Zellwänden und Membranen von Eukarionten, die zur Vernichtung von allen Pilzen, Bakterien, Viren, Sporen und Hefen führt und die den Tracheen -Atmungsaparat von Insekten verschliesst, lahm legt und folglich zu deren Tod führt.

Ozon kann die mehrschichtige Zellwandstruktur von Pflanzen nicht auflösen. Ozon kann somit nicht in die Pflanzen eindringen, ausser unter Langzeit Einwirkung durch die Atmungs Stomata.

Ozon wirkt im kurzen Kontakt Prinzip und hat keine Systemische Wirkung.

Ozon hinterlässt keine Rückstände, da es in kürzerster Zeit wieder in Sauerstoff zerfällt.

Ozon hinterlässt wegen seiner kurzen Einwirkungsdauer keine Schäden auf Pflanzen.

Ebenfalls verursacht Ozon keine Resistenzen bei Mikroben oder pflanzlichen Schädlingen.

Ozon ist deshalb ein äusserst effektives Biocid gegen alle Pilze, Bakterien, Viren, Hefen, Biofilme, Protozoen und höhere Lebewesen wie kleine Insekten, Würmer, Spinnmilben und andere Schad - Parasiten.

Ozon ist seit dem 26. Juni 2001 von der Food and Drug Administration FDA in den USA als Zusatzstoff im Direktkontakt mit Lebensmitteln gestattet.

Ozon ist ebenfalls als Desinfektions - Produkt von der EPA (Environmental Protection Agency) in den USA gestattet.

UV - C Licht, als elektromagnetische Direkt - Strahlung hat im Wellenbereich von 254 Manometer optimale biocide Wirkung gegen Bakterien, Hefen, Viren und Insekten.

Die Strahlung bewirkt bei Mikroorganismen die Auflösung von Zell - Membranen und zerstört DNS Strukturen.

Im Wellenbereich von 185 Nanometer erzeugt UV -C Strahlung Ozon, welches unter hoher Luftfeuchtigkeit hoch oxidative labile Hydroxilverbindungen erzeugt, die die Wirkung von Ozon als Biocid wesentlich verstärken. (mikrobielles Hürde - Prinzip) UV-C Bestrahlung von Lebensmitteln ist seit 1997 von der FDA in den USA zugelassen und für pflanzliche Lebensmittel ebenfalls in der Deutschen Strahlenschutzverodrnung.

Für die Effizienz der UV-C Strahlung ist die Strahlen Dosis entscheidend, die in mW/sec/cm 2 (Milli Watt pro Sekunde pro Quadrat- Centimeter bestrahlter Fläche) angegeben wird.

Die Dosis zur Eliminierung von Mikroorganismen beträgt vorzugsweise 4'000 bis 15'000 mW/sec/cm 2 (abhängig von Art)

Für Schadinsekten ca. 500'000 -1'500'000 mW/sec (abhängig von Art).

Der Erfinder hat in 10-jähriger Forschungsarbeit im Labor und in Feldversuchen die Effizienz des neuen Pflanzenschutz - Verfahrens mit Ozon - Wasser und UV - Bestrahlung und der entsprechenden Aplikations - Technologie bestätigt.

Das neue Pflanzenschutz -. Verfahren erreichte in statistisch relevanten Feldversuchen 92% Effizienz in Mischinfektionen und gleichzeitigem Befall von 3 Insekten Arten in Weizen und Buschbohnen, Tomaten und Gurken im Freiland Anbau und im Gewächshaus. Andere kulturen, in denen die Erfindung getestet wurde und die präventiv behandelt wurden, zeigten keine Ertrags mindernde Schäden.

Nach Kennnisstand des Erfinders sind bis heute keine wissenschaftlichen Arbeiten auf dem Gebiet des Pflanzenschutzes mit Ozon und UV - Bestrahlung und mit der entsprechenden Aplikations -Technik für den Feldeinsatz veröffentlicht.

### DIE LÖSUNG DER AUFGABE

Die Lösung der Aufgabe ist durch die Mekmale der unabhängigen Patent -Ansprüche definiert.

Gemäss der Erfindung weist ein Verfahren zur Anwedung im Pflanzenschutz gegen schädliche Pilze, Hefen, Bakterien, Viren, Sporen und Schadinsekten die Art und Weise der Biocide (Ozon) und UV-C - Strahlung und dessen Herstellung auf und die Art und Weise der Applikations -Technologie für einen effizienten Einsatz.

Die Erfindung bildet ein integriertes System in welchem die technischen Komponenten zur Ozon- und UV-Erzeugung und die Aplikations -Technologie in Form einer Traktor gezogenen, oder Traktor Hydraulik getragenen Sattelspritze oder Rücken getragene Handfeldspritze integriert sind.

Dabei liegt der Schwerpunkt der Innovation nicht nur in der Kombination von Ozon und UV-C - Strahlung als neue Anwendung und Hürden - Technologie zur Schädlingsbekäpfung im Pflanzenschutz, sondern auch in den kombinierten neuen Aplikationstechniken, dem Dipol elektrischen Spray - Verfahren und der Air Jet Spray Technik.

Die neue kombinierte Applikationstechnik ist essenziell für den erfolgreichen Einsatz von Ozon und UV-C-Bestrahlung und erfüllt alle Parameter zur optimalen Wirkungsweise des neuen Pflanzenschutz Verfahrens.

Ohne die spezielle Anwendungstechnik ist die Wirkung von ozoniertem Wasser und UV-C - Strahlung ungenügend, da die Wirkungsdauer von Ozon wegen der hohen Molekukar-Instabilität sehr volatil und nur sehr kurz ist.

Weiter ist die Erfindung innovativ bezüglich des Verfahrens zur Ozonierung des Wassers mittels eines Venturi Ventils und nachfolgender Injektionstourbine, die speziell für diesen Anwendungszweck konstruiert wurden.

Mit dieser neuen Injektions - Technik, der innigen Vermischung von Ozon mit dem Spray - Wasser, in Kombination mit einem anorganischen Netzmittel kann die Ozonkonzentration im Wasser bis ca. 17% betragen und hat eine dreifache Verbleibdauer (bis 1 Stunde) im Wasser, ohne dass sich OzonGas in Sauerstoff umwandelt. Damit kann der Wirkungsgrad von Ozon als Biocid wesentlich gesteigert werden.

Die Erfindung des neuen Pflanzenschutz - Verfahrens besteht in der Kombination von Ozon - Wasser (Biocid) Erzeugung, Tank - Zwischenlagerung des Ozon - Wassers und Ausbringung des Biocids mittels Dipol elektrischer Spray Technologie und direkter UV-C - Bestrahlung (Biocid).

Das neue Anwendungs - Verfahren für den Pflanzenschutz mit Ozon haltigem Wasser und UV-C - Bestrahlung besteht aus folgenden technischen Komponenten:
1.Vorspritz - Einrichtung, vorugsweise Zapfwellen oder elektrisch betrieben, vorzugsweise in Form einer Balken - Sattelspritze mit Tank, zur Vorbenetzung der Pflanzen mit negativ geladenem Wasser, das ein anorganisches Netzmittel enthält, mittels am Balken angebrachten Wasser - SprayDüsen und Air - Jet Wirbelstrom Düsen, UV-C - Strahler -Lampen und Spritz - Balken Abdeckung zur exakten Einstellung des Spray Strahls und der UV-C- Bestrahlung.
   Die Vorspritz - Einrichtung enthält folgende technische Hauptteile:
   1 A 3 - Punkt - Tragrahmen mit Chassis und Tank - Halterung
   2 A Frontzapfwellen- Anschluss oder Elektro Motor
   3 A Tank für Wasserfüllung
   4. A Spritz - Druckpumpe
   5 A 2 Druck Manometer einstellbar (Eingangs- Ausgangs- Druck)
   6 A 2 Laterale Teleskop Spritz - Balken mit 20 UV-Lampen 35W
   7 A 1 Anode
   8 A 1 Transformer für Wechsel- und Gleichstrom zur minusAufladung des Spritzwassers
   9 A 1 Luftgebläse oder Luftkompressor mit Drucktank zur Erzeugung des Luftwirbelstroms
   10 A Diverse Spritzdüsen für Wasser und Luft
   11 A 1 Wasser Dosierventil
   12 A 1 Druckumkehr-Ventil für Wasser- Mixer im Tank mit Hahn
   13 A 1 Einfüllstutzen für Wasser mit Druck dichter Abdeckung
   14 A 1 Tank Drainage - Ventil - Hahn
   15 A 1 Spritzbalken - Abdeckung, einstellbar zur präzisen Spritz- und Wirbelstrom und UV - Licht - Führung.
   16 A 1 Zapfwellenanschluss für Spritz Druckpumpe
2. Gezogene oder hydraulisch getragene Teleskop - Balken Sattelspritze mit Zapfwellen- oder elektrischem Antrieb zur Ausbringung von Ozon - Wasser über Wasser Spray Düsen mit Jet Air -Wirbestrom - Technik mittels Spezieller Luftdüsen und UV-C - Lampen und Spritz - Balken - Abdeckung zur exakten Einstellung des Spray- und Luft - Strahls und der UV - Bestrahlung.
   Die Ozon Wasser Spray Einrichtung enthält folgende technische Hauptteile:
   1 B 3-Punkt Tragrahmen mit Chassis und Tankhalterung
   2 B 1 Elektro Generator mit Schaltschrank
   3 B 1 Luftsperator im Schwingsiebprinzip zur Erzeugung von Sauerstoff
   4 B 1 Ozongas - Erzeuger mittels Corona Kathoden - Entladung (Erzeugung aus 98% Sauerstoff)
   5 B 1 Ozon Gas Dosierventil mit Hahn
   6 B 1 Ozon Gas in Wasser - Messgerät
   7 B 1 Venturi Ventil zur Injektion von Ozon ins Wasser
   8 B 1 Elektrisch betriebene Druckpumpe zum Wassertank
   9.B 2 Druck Manometer (Eingang- Ausgang Druckpumpe mit 2 Ventil Hahnen.
   10 B Tourbinen Ozon -Gas Mixer
   11 B 1 Wasser Tank für Ozon haltiges Wasser
   12 B 1 Fahrbares Chassis oder Aufhängevorrichtung für Traktor -Hydraulik
   13 B 1 Zapfwellen- oder elektrisch getriebene Wasser Druck - Pumpe aus rostfreiem Stahl für die Ausbringung von Ozon - Wasser.
   14 B 2 Laterale Teleskop Spritz - Balken mit min. 20 angebrachten 35 W UV - Lampen.
   15 B Diverse Ozon Wasser Spray Düsen und spezielle Air - Jet Wirbelstrom Düsen.
   16 B 1 Zapfwellen oder elektrisch betriebenes Luftgebläse oder Luftkompressor mit Druck- Tank zur Erzeugung des Luftwirbelstroms via Düsen.
   17 B 1 Wasserdosierventil
   18 B 2 Druckmanometer (Eingangs- Ausgangsdruck)
   19 B 1 Druckumkehr Ventil für Wassermixer im Tank
   20 B 1 Einfüllstutzen für Wasser in den Tank mit druckdichter Abdeckung.
   21. B 1 Tank Drainage - Ventil - Hahn
   22 B 1 Spritzbalken - Abdeckung, einstellbar zur präzisen Sparay- und Wirbelstrom - Führung und UV - Bestrahlung.
3. Zug- und Traggerät

Traktor (min. 65 HP) mit Front und Heck Hydraulik, mit einstellbarem Front - und Heck - Zapfwellenantrieb, mit geschlossener Fahrer - Kabine mit Überdruck Ventilations - Belüftung und Ozonfilter.

Das neue Anwendungsverfahren der Erfindung beinhaltet zwei wesentliche Schritte:
1.Umfassende Vor- Benetzung der zu behandelnden Pflanzen mit negativ aufgeladenem Wasser und mittels eines anorganischen Netzmittels mit Hilfe der Dipol elektrischen Luftwirbelstrom Technik (Air Jet) und UV - Bestrahlung mittels UV-C Lampen 35 W.

Diese Vorbehandlung der Pflanzen ist notwendig, damit in der unmittelbar nachfolgenden Ozon - Wasser Spritz - Behandlung alle Pflanzenteile mit Ozon in Kontakt kommen und sich das Ozon - Wasser regelmässig in Form eines Films auf dem Blattwerk verteilt.

Durch den Wirbelstrom der Luft aus den Luftdüsen am Spritz - Balken werden die Pflanzen, ohne Schaden zu nehmen, so verwirbelt, dass auch die Unterseite des Blattwerkes und alle Teile im Zentrum und am Fuss der Pflanzen benezt werden.

Das anorganische Netzmittel garantiert eine Verteilung des Wassers auf den Nutzpflanzen in Form eines Fims, gebildet aus negativ geladenem Wasser und nicht als Mikro - Tropfen, was eine gute Benetzung garantiert.

Die negative elektrische Ladung des Wasserfilms garantiert, dass das unmittelbar danach ausgebrachte Ozon - Wasser mit positivem Dipol in Sprayform an alle Teile und Zonen des Pflanzenblattwerkes gelangt.

2. Spritzbehandlung der Pflanzen mit Ozon - Wasser, mittels neuer Ozon - Spritzventil Mischtechnik, respektive mittels Venturi Ventil und Drucktourbine und Air - Jet Luftwirbelstrom Technik und UV-C - Licht Bestrahlung.

Das aus Sauerstoff (im Luftseperator hergestellter 98% Sauerstoff) mittels Corona - Kathoden - Entladung erzeugte Ozon wird bei einem Druck von vorzugsweise 1.5 bis 2.5 bar Druck mittels eines Venturi Ventils in das zum Wasser - Tank führende Wasser der Balkenspritze im Saugprinzip injektiert und das Ozon haltige Wassergemisch in der Folge in einer speziell konzipierten Drucktourbine bei vorzugsweise 4.7 bis 6 bar Druck so verwirbelt, dass die Ozon - Gas -Teile im Wasser in Mikroform vorliegen und sich mit dem Wasser in der gewünschten Konzentration gut verbinden.

Dieses neue Verfahren verhindert eine rasche Ausgasung des Ozons und garantiert, dass das Ozon - Gas beim Spray - Vorgang bei vorzugsweise 4 bis 10 bar Druck in der Wasserlösung verbleibt.

Gespritzt wird vorzugsweise mit 4 bis 10 bar Druck je nach Pflanzenart und Applikation.

Dabei kommen verschiedene Spray- und Luft - Düsen zum Einsatz.

Die Luft - Düsen bewirken mit der speziellen, individuell einstellbaren Spritzbalken - Abdeckung einen Luftwirbel, der die zu behandelnden Pflanzen so verwirbelt, dass das Ozon haltige Wasser an alle Pflanzenteile und vor allem auch an die Unterseite, das Herz und den Fuss des Pflanzenblattwerkes gelangt.

Die vorhergehende Benetzung mit negativ elektrisch geladenem Wasser bewirkt, dass sich das Ozon mit positivem Dipol sofort an negative Wasser - Moleküle im Wasser - Film der Vorbenetzung anhaftet und seine oxidative Wirkung vollumfänglich auf allen Teilen der Pflanzen gleichzeitig und mit der gleichen Wirkung ausübt.

Durch den intensiven Kontakt mit Ozon - Wasser, mit einer Konzentration von Vorzugsweise 2mg/liter bis 40 mg/Liter in der Spritzlösung (abhängig vom Schädlingsdruck und Pflanzenart) werden Pilze, Hefen, Bakterien, deren Sporen und Viren, Protozoen und Insekten abgetötet.

Dabei spielen der KM Faktor (Konzentration in mg/ Liter der Lösung mal Einwirkzeit in Minuten) eine entscheidende Rolle.

Jeder Pflanzenschädling hat einen spezifischen KM - Faktor, der vorzugsweise zwischen ca 10 und 200 mg Min. liegt.

Entsprechend dem zu erwartenden Schädlingsbild und Schädlingsdruck wird die Ozon - Wassekonzentration eingestellt.

Die Wirkungsdauer von Ozon beträgt 20 Sekunden bis ca. 20 Minuten.

Die Halbwertszeit von Ozon in Wasser ist ca. 2 Minuten, d.h. alle 2 Minuten reduziert sich die Konzentration von Ozon in Wasser um die Hälfte.

Das Ozon Gas verflüchtigt sich rasch in Form von atomarem Sauerstoff.

Die Bestrahlung mit UV-C- Licht mittels Lampen mit einer Leistung von vorzugsweise 35 Watt und einer Wellen - Länge von 254 Nanometer und / oder 185 Nanometer hat mit der Bildung von hochoxidativen Hydroxyl - Verbindungen im feuchten Milieu des Spraynebels einen zusätzlichen biociden mikrobiellen Hürden - Effekt.

Der Einsatz von Ozon haltigem Wasser in Kombination mit UV - Licht im Pflanzenschutz ist äusserst effektiv, billig und Umwelt freundlich und kann auch im ökologischen oder Bio - Landbau eingesetzt werden.

Der Effizienz - Grad hat in eigenen Feldversuchen bis 92% betragen, was die Normen in der konventionellen chemischen Pflanzenschutz - Technik erfüllt oder sogar übersteigt.

Ozonbehandlung in Kombination mit UV-C - Licht im Pflanzenschutz erzeugt keine Rückstände.

Alles Ozon wandelt sich in kurzer Zeit (Stunden) wieder in Sauerstoff um.

Ozon und kurz einwirkendes UV-C - Licht beeinträchtigen die Pflanzen in keiner Weise, da Ozon und UV - Licht nur in Mikroben - Zellwände und nicht in Pflanzenzellen eindringen können, da die Kontakt - Zeit zu kurz ist.

Ozonbehandlungen und UV-C - Bestrahlung im Pflanzenschutz erzeugen keine Resistenzen bei Schädlingen, da die Wirkungsweise der Biocide ausschliesslich auf oxidativen Vorgängen beruht.

Ozonbehandlung im Pflanzenschutz ist Umwelt freundlich, da alles erzeugte Ozon wieder in Sauerstoff zerfällt.

Die Anwendung von Ozon in Kombination mit UV - Licht ist wesentlich billiger als herkömmliche chemische Spritzverfahren, da keine teuren Chemikalien verwendet werden müssen.

Die Investitionen in die Hardware für das Spritzverfahren sind ca. doppelt so gross wie konventionelle Spritz - Maschinen. Die durchschnittliche Amortisationsdauer eines Ozon Technik Spritz - Gerätes ist ca. 4 Jahre.

Die Lebensdauer min. 10 bis 15 Jahre.

In dieser Periode können mehr als ¾ der Spritzkosten im Vergleich zur konventionellen chemischen Behandlung eingespart werden.

### AUSFÜHRUNG DER ERFINDUNG

Zur Ausführung des Verfahrens zur Kontrolle von Pilz-, bakterien-, Viren und Insektenbefall im Pflanzenschutz mittels Dipol elektrischer Air Jet Spray - Technik, Ozon haltigem Wasser und UV-C - Bestrahlung sind vorzugsweise zwei Sattel - Spritzen mit Teleskop Spritz - Balken oder einer gezogenen Tourbinen -Spritze, wie sie im Obstbau oder Weinbau eingesetzt wird, nötig.

Die Spritzen werden an der Heck- und Front - Hydraulik oder der Anhängevorrichtung mit einem Traktor, mit einer Motorenleistung von vorzugsweise 65 PS und mehr verbunden.

In beiden Fällen wird der Tank je nach Ausführung mit 500 bis 2000 Liter normalem, sauberem, nicht zu Kalk haltigem Wasser gefüllt.

Die beiden Zapfwellen zum Antrieb der Spritzen -Druckpumpen und den Luftgebläsen oder Druckluft Kompressoren werden an den Zapfwellen -Antrieben des Traktors montiert (Heck und Front).

Der mit Benzinmotor angetriebene Strom - Generator, mit vorzugsweise min. 8 KWA Leistung, wird in Betrieb gesetzt.

Die Kabel zum Stromtransformer auf der Sattel - Spritze an der Front - Hydraulik ans Stromnetz im Schaltschrank angeschlossen.

Der Transformator wird auf Gleichstrom geschaltet, mit einer Spannung von vorzugsweise 1000 - 2000 Volt.

Der Stromfluss ist jetzt über die Anode im isolierten Spritztank hergestellt, und das Wasser und das Netzmittel werden mit einer negativen Ladung versehen, die nötig ist, um bei der Vorbenetzung auf den Pflanzen - Blättern einen negativ geladenen Wasser- Film zu bilden.

Das Vorbenetzungswasser wird mit einem anorganischen Netzmittel, vorzugsweise als 2% Lösung versetzt.

Der Sauerstoff Luftseperator und das Ozonersugungsgerät mit Corona Kathoden Entladung werden in Betrieb gesetz.

Ozon wird jetzt produziert und mit einem Druck von vorzugsweise 1,5 bis 2,5 bar mit einem Venturi Ventil im Saug - Prinzip in das von einer Druckpumpe mit vorzugsweise 5 bis 8 KWA Leistung angetriebene Wasserzirkulationssystem der Heck - Sattelspritze injektiert. Dabei passiert das unter Druck stehende Ozon - Wasser den im Kreislauf integrierten Tourbo - Mixer, der das Ozon in Mikroform an Wassermoleküle bindet und zu einer verbesserten Vermischung und stärkeren Verbindung des Ozons mit dem Wasser führt.

Nach ca. 5 Minuten ist die gewünschte und eingestellte Ozon-Konzentration im Tank erreicht, und das Ozonerzeugungsgerät schaltet automatisch auf O - Betrieb.

Falls die eingestellte Ozon - Konzentration im Spritz - Tank abfällt, schaltet sich das Ozon - Gerät automatisch wieder ein. Dieser Automatismus wird mit einer Ozon - Mess -Sonde und einer elektrischen Steuerung geregelt, die sich im Elektro - Schaltschrank befinden.

Die UV-C - Lampen sind ans Stromnetz angeschlossen und mit Schaltknopf bedienbar.

Die Spritzgeräte sind jetzt Einsatz bereit.

Die Zapfwellen -Antriebe der Front- und Heckspritze werden eingeschaltet.

Der Spritzdruck und der Wirbelstrom - Luft -Druck,vorzugsweise 4 bis 10 bar, je nach Anwendung, bauen sich auf.

Mit ausgefahrenen Höhe getrimmten Spritzbalken und korrekt eingestelltem Spritzdruck und Spritzmenge, vorzugsweise ca. 30 - 80 Liter / Minute, kann der Spritz - Vorgang beginnen.

Mit der Sattelspritze an der Front - Hydraulik des Traktors wird das elektrisch negativ aufgeladene Vornetz -.Wasser mit dem Netzmittel (ca. 2% in der Lösung) mittels der Zapfwellen getriebenen Spritzpumpe (5 - 8PS) über die speziellen Spritzwasser-Düsen auf die zu behandelnden Pflanzen gesprüht, und gleichzeitig werden die Pflanzen durch die aus den speziellen Luft - Düsen, mittels Luftgebläse oder Druckluft - Kompressor erzeugte, austretende Druck-Luft, im Schutze der Spritzbalken - Abdeckung, so verwirbelt, dass der Spray - Nebel alle Pflanzenteile, inklusive Unterseite, Herz und Fusspartien voll benetzen kann.

Ein negativ geladener Wasserfilm bildet sich auf allen Pflanzenteilen.

Nach wenigen Sekunden erfolgt die Ozon - Spritzung aus der Heck - Spritze des fahrenden Traktors (Geschwindigkeit ca. 3 bis 5 km/h.

Die Ozon haltige Spritzbrühe (milchiges Aussehen) wird im gleichen Verfahren der Zapfwellen getriebenen Druckpumpe über die speziellen Spraydüsen mit vorzugsweise 4 bis 10 bar Druck (nach Pflanzen- und Schädlingsart verschieden) über die mit negativ elektrisch geladenen, vorbenetzten Pflanzenteile gesprüht.

Dabei wird gleichzeitig mit Druckluft aus den Luftdüsen im Schutze der Spritzbalken -Abdeckung eine Verwirbelung des Pflanzgutes angestrebt, damit das Ozon haltige Wasser, vorzugsweise mit einer Konzentration von 3 bis 40 mg/Liter, mit allen Teilen der Pflanzen in Kontakt kommt.

Durch die negative Ladung des Vornetz - Wasserfilms ergibt sich mit dem Ozon - Spray - Nebel mit positivem Dipol - Charakter eine innige elektrostatische Verbindung an allen Stellen der Pflanzen, was den oxidativen Wirkungsgrad des Ozons erhöht und garantiert, dass die Oxidationswirkung an Schädlingen Flächen deckend gewährleistet ist.

Die an den Spritzbalken der Front- und Heck - Spritze angebrachten Ultra Violett - Strahler verstärken, durch die Bildung von hochoxidativen Hydroxil Verbindungen, gebildet in der feuchten Atmosphäre des Spray - Nebels, den Wirkungs - effekt des Ozon - Einsatzes beträchtlich. Das mikrobiologische Hürden - Prinzip, wonach Selbstschutz Mechanismen bei Mikroben bei gleichzeitiger Anwedung von mehreren Biociden versagen, funktioniert ausgezeichnet.

Mit dieser neuen Spray - Technik und dem Einsatz von ozoniertem Wasser ist es möglich, 92% aller Pilze, Hefen, Batterien, Viren und Insekten zu vernichten.

Die beschriebene Erfindung ist ein neues bahnbrechendes Verfahren der Spritztechnik im Pflanzenschutz.

Sie verbindet alle Ansprüche eines Zeit gemässen, modernen Pflanzenschutzes und kann auch im ökologischen und Bio - Pflanzenbau angewendet werden.

Das neue Spritz - Verfahren und die angwendete Technik sind einfach, billiger, Umwelt freundlich und hinterlassen keine schädlichen Rückstände auf Nahrungspflanzen und erzeugen keine Resistenzen bei Pilzen, Hefen, Viren und Insekten, wie das bei herkömmlichen Chemie - Verfahren bekannt ist.

### Zug_- und Traggerät

Traktor (min. 65 HP) mit Front und Heck Hydraulik, mit einstellbarem Front - und Heck- Zapfwellenantrieb, mit geschlossener Fahrer - Kabine mit Überdruck Ventilations - Belüftung und Ozonfilter.

Die Vorspritz - Einrichtung enthält folgende technische Hauptteile:
1 A 3 - Punkt - Tragrahmen mit Chassis und Tank - Halterung
2 A Frontzapfwellen - Anschluss oder Elektro Motor
3 A Tank für Wasserfüllung
4 A Spritz - Druckpumpe
5 A 2 Druck Manometer einstellbar (Eingangs- Ausgangs- Druck)
6 A 2 Laterale Teleskop Spritz - Balken mit 20 UV-Lampen 35W
7 A 1 Anode
8 A 1 Transformer für Wechsel- und Gleichstrom zur minusAufladung des Spritzwassers
9 A 1 Luftgebläse oder Luftkompressor mit Drucktank zur Erzeugung des Luftwirbelstroms
10 A Diverse Spritzdüsen für Wasser und Luft
11 A 1 Wasser Dosierventil
12 A 1 Druckumkehr-Ventil für Wasser- Mixer im Tank mit Hahn
13 A 1 Einfüllstutzen für Wasser mit Druck dichter Abdeckung
14 A 1 Tank Drainage - Ventil - Hahn
15 A 1 Spritzbalken - Abdeckung, einstellbar zur präzisen Spritz- und Wirbelstrom und UV - Licht - Führung.
16 A 1 Zapfwellenanschluss für Spritz Druckpumpe

Die Ozon Wasser Spray Einrichtung enthält folgende technische

### Hauptteile:

1 B 3-Punkt Tragrahmen mit Chassis und Tankhalterung
2 B 1 Elektro Generator mit Schaltschrank
3 B 1 Luftsperator im Schwingsiebprinzip zur Erzeugung von Sauerstoff
4 B 1 Ozongas - Erzeuger mittels Corona Kathoden - Entladung (Erzeugung aus 98% Sauerstoff)
5 B 1 Ozon Gas Dosierventil mit Hahn
6 B 1 Ozon Gas in Wasser - Messgerät
7 B 1 Venturi Ventil zur Injektion von Ozon ins Wasser
8 B 1 Elektrisch betriebene Druckpumpe zum Wassertank
9.B 2 Druck Manometer (Eingang- Ausgang Druckpumpe mit 2 Ventil Hahnen.
10 B Tourbinen Ozon -Gas Mixer
11 B 1 Wasser Tank für Ozon haltiges Wasser
12 B 1 Fahrbares Chassis oder Aufhängevorrichtung für Traktor -Hydraulik
13 B 1 Zapfwellen- oder elektrisch getriebene Wasser Druck - Pumpe aus rostfreiem Stahl für die Ausbringung von Ozon - Wasser.
14 B 2 Laterale Teleskop Spritz - Balken mit min. 20 angebrachten 35 W UV - Lampen.
15 B Diverse Ozon Wasser Spray Düsen und spezielle Air- Jet Wirbelstrom Düsen.
16 B 1 Zapfwellen oder elektrisch betriebenes Luftgebläse oder Luftkompressor mit Druck - Tank zur Erzeugung des Luftwirbelstroms via Düsen.
17 B 1 Wasserdosierventil
18 B 2 Druckmanometer (Eingangs- Ausgangsdruck)
19 B 1 Druckumkehr Ventil für Wassermixer im Tank
20 B 1 Einfüllstutzen für Wasser in den Tank mit druckdichter Abdeckung.
21. B 1 Tank Drainage - Ventil - Hahn
22 B 1 Spritzbalken -Abdeckung, einstellbar zur präzisen Sparay- und Wirbelstrom - Führung und UV - Bestrahlung.

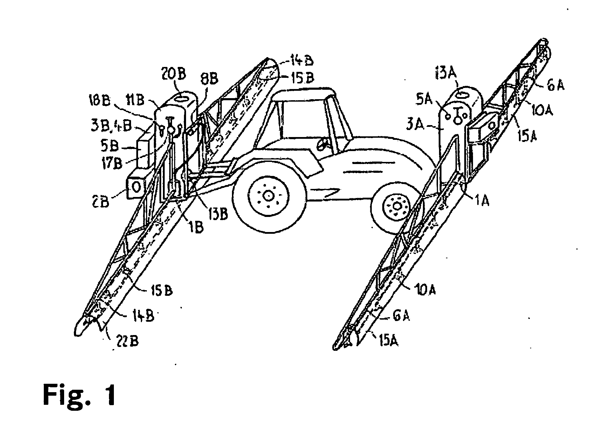

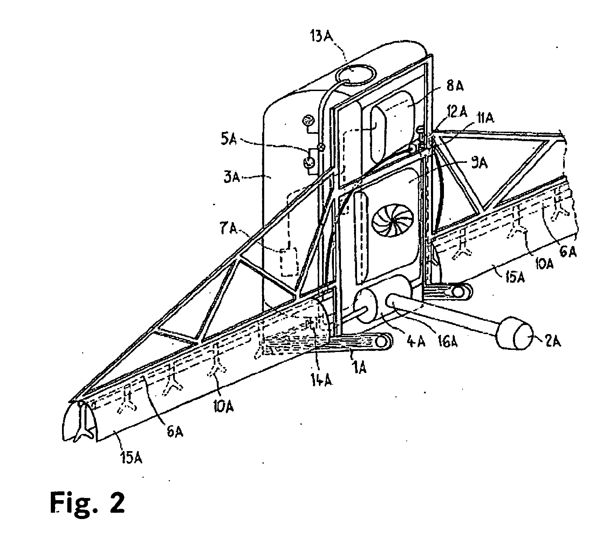

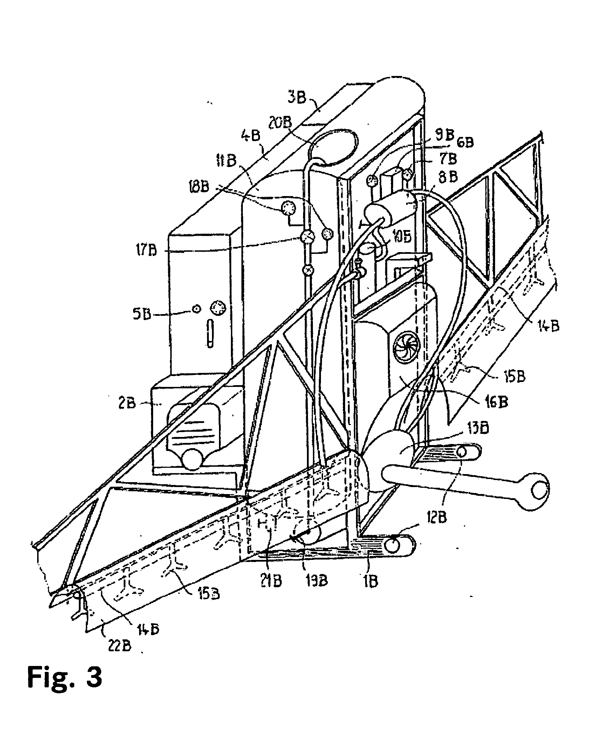

## Patentansprüche

1. Verfahren im Pflanzenschutz zur Kontrolle von Pilz-, Hefen-, Bakterien-, Virus- und Insekten-Befall, **dadurch gekennzeichnet, dass** in einem ersten Schritt Pflanzen mittels dipol-elektrischer Air-Jet-Spray technik mit anorganischem Netzmittel benetzt und mit Ultraviolett-C-Licht bestrahlt werden und in einem zweiten Schritt mittels dipol-elektrischer Air-Jet Spray technik mit ozonhaltigem Wasser besprüht und mit Ultraviolett-C-Licht bestrahlt werden.

2. Verfahren gemäss Anspruch 1, **dadurch gekennzeichnet, dass** zwei Spritzgeräte verwendet werden.

3. Verfahren gemäss Anspruch 2, **dadurch gekennzeichnet, dass** mit einem ersten Spritzgerät alle Pflanzenteile zuerst mit elektrisch aufgeladenem Wasser und Netzmittel angefeuchtet werden.

4. Verfahren gemäss einem der Ansprüche 2 oder 3, **dadurch gekennzeichnet, dass** mit einem zweiten Spritzgerät alle Pflanzenteile mit dipoligem ozoniertem Wasser besprüht werden.

5. Verfahren gemäss einem der Ansprüche 1 bis 4 **dadurch gekennzeichnet, dass** das Anfeucht spraywasser aus einem ersten Tank und das ozon haltige Spraywasser aus einem zweiten Tank mittels Air-Jet-Luftwirbelstrom aus speziellen Luftdüsen, erzeugt durch eine Luftturbine, ein Luftgebläse oder einen Luftkompressor, verwirbelt auf alle Pflanzenteile gesprüht wird.

6. Vorrichtung zur Durchführung des Spritzverfahrens gemäss einem der Ansprüche 1 bis 5, **dadurch gekennzeichnet, dass** die Vorrichtung durch dipolelektrische 1 Spraytechnologie betreibbar ist und UV-C-Lampen umfasst sowie Spraydüsen und Air-Jet-Wirbelstrom düsen zur Benetzung mit anorganischem Netzmittel und Spraydüsen mit Air-jet-Wirbelstrom Technik zur Ausbringung von Ozonwasser umfasst.

7. Vorrichtung gemäss Anspruch 6, **dadurch gekennzeichnet, dass** sie aus zwei Spritz geräten besteht.

8. Vorrichtung nach Anspruch 7, **dadurch gekennzeichnet, dass** ein erstes der beiden Spritzgeräte zur Vorbenetzung der Pflanzen mit elektrisch negativ aufgeladenem Wasser mit Netzmittel dient und folgende technische Hauptteile enthält:
- 3-Punkt montierbares Chassis mit Rahmen und Tankhalterung
- isolierter Wassertank
- Elektrischer Transformer
- Anode im Wassertank mit Anschluss kabel und Sicherung
- Laterale Teleskopspritzbalken mit speziellen Luft- und Wasserdüsen inklusive Leitungen
- UV-C-Lampen mit elektrischen Leitungen
- Wasserdruckpumpe mit Druckventilen und Manometern, Steuerung und Hahnen, verbunden durch Leitungen mit dem Wassertank und den Spritzbalken
- Luftgebläse oder Luftkompressor mit Steuerung, verbunden mit Leitungen zu den Luft-Düsen an den Spritzbalken, zur Verwirbelung des Spraynebels.
- Antriebswelle oder Elektroantrieb
- Umkehrdruck-und Drainageventile mit Hahn
- Spritzbalkenabdeckungen, einstellbar.

9. Vorrichtung nach Anspruch 7, **dadurch gekennzeichnet, dass** ein zweites der beiden Spritzgeräte zum Ausbringen von ozoniertem Wasser dient und folgende technische Hauptteile enthält:
- 3-Punkt-montierbares Chassis mit Rahmen und Tankhalterung, oder gezogenes Anhängerchassis
- Isolierter Wassertank
- Laterale Teleskopspritzbalken mit speziellen Luft- und Wasserdüsen inklusive Leitungen oder Wassersprayturbine
- UV-G-Lampen mit elektrischen Leitungen
- Wasserdruckpumpe mit Druckventilen und Manometern, Steuerung, und Hahnen, verbunden durch Leitungen mit dem Wassertank und den Spritzbalken
- Luftgebläse oder Luftkompressor mit Steuerung, verbunden mit Leitungen zu den Luftdüsen an den Spritzbalken, zur Verwirbelung des Spraynebels.
- Antriebswelle oder Elektroantrieb
- Umkehrdruck-und Drainageventile mit Hahn
- Sprtitzbalkenabdeckung, einstellbar
- Elektrogenerator mit Schaltschrank
- Ozonwasserpumpe
- Luftseparator zur Gewinnung von Sauerstoff
- Ozonerzeuger mit Corona Kathodenentladung
- Venturiventil
- Ozonturbinenmixer
- Ozongasdosierer
- Ozonkonzentrationsmessgerät.

10. Vorrichtung gemäss einem der Ansprüche 7 bis 9 **dadurch gekennzeichnet, dass** beide Spritzgeräte an den Spritzbalken auf der gesamten Länge mit UV-C "Lampen ausgerüstet sind, die zusätzlich zur elektromagnetischen Direktbestrahlung, im Spraynebel hoch oxidative Hydroxilverbindungen erzeugen, die eine effiziente biocide Wirkung haben und im Hürdenbrinzip die biocide Wirkung der Ozonbehandlung verstärken

11. Vorrichtung gemäss einem der Ansprüche 6 bis 10 **dadurch gekennzeichnet, dass** alle Spritzbalken mit einer adaptierbaren Spritzbalken Abdeckung so ausgestattet sind, dass der Sprayvorgang, respektive die Richtung und Wirkung der Sprayapplikation und der UV-C-Licht Bestrahlung je nach Bedarf eingestellt werden können.

12. Biocide Anwendung des Verfahrens im Pflanzenschutz gemäss einem der Ansprüche bis 5 zum Schutz und zur Vernichtung von Pilzen,Hefen, Bakterien, Viren, Sporen, Insekten und anderen Schädlingen und deren Gelege auf Nutzpflanzen.

## Claims

1. Method in plant protection for controlling attacks by fungi, yeast, bacteria, viruses and insects, **characterized in that** plants are, in a first step, wetted with an inorganic wetting agent using dipole electric air jet spray technology and irradiated with ultraviolet C light and, in a second step, sprayed with ozone-containing water using dipole electric air jet spray technology and irradiated with ultraviolet C light.

2. Method according to Claim 1, **characterized in that** two spray devices are used.

3. Method according to Claim 2, **characterized in that**, with a first spray device, all plant parts are first wetted with electrically charged water and wetting agent.

4. Method according to one of Claims 2 or 3, **characterized in that**, with a second spray device, all plant parts are sprayed with dipole ozonated water.

5. Method according to one of Claims 1 to 4, **characterized in that** the wetting spray water is sprayed from a first tank and the ozone-containing spray water is sprayed from a second tank by means of an air jet air eddy current from special air nozzles, generated by an air turbine, an air blower or an air compressor, sprayed with an eddying effect onto all plant parts.

6. Apparatus for carrying out the spray method according to one of Claims 1 to 5, **characterized in that** the apparatus is operatable by dipole electric spray technology and comprises UV-C lamps and comprises spray nozzles and air jet eddy current nozzles for wetting with an inorganic wetting agent and spray nozzles with air jet eddy current technology for applying ozone water.

7. Apparatus according to Claim 6, **characterized in that** it consists of two spray devices.

8. Apparatus according to Claim 7, **characterized in that** a first one of the two spray devices serves for pre-wetting the plants with electrically negatively charged water with wetting agent and contains the following technical main parts:
- 3-point mountable chassis with frame and tank holder
- isolated water tank
- electric transformer
- anode in the water tank with connecting cable and securing means
- lateral telescope spray boom with special air and water nozzles including lines
- UV-C lamps with electric lines
- water pressure pump with pressure valves and manometers, controller and taps, connected by lines to the water tank and the spray booms
- air blower or air compressor with controller, connected by lines to the air nozzles on the spray booms for eddying the spray mist
- drive shaft or electric drive
- reverse pressure and drainage valves with tap
- spray boom covers, adjustable.

9. Apparatus according to Claim 7, **characterized in that** a second one of the two spray devices serves for applying ozonated water and contains the following technical main parts:
- 3-point mountable chassis with frame and tank holder, or drawn trailer chassis
- isolated water tank
- lateral telescope spray boom with special air and water nozzles including lines or water spray turbine
- UV-C lamps with electric lines
- water pressure pump with pressure valves and manometers, controller and taps, connected by lines to the water tank and the spray booms
- air blower or air compressor with controller, connected by lines to the air nozzles on the spray booms for eddying the spray mist
- drive shaft or electric drive
- reverse pressure and drainage valves with tap
- spray boom covers, adjustable
- electric generator with switch box
- ozone water pump
- air separator for production of oxygen
- ozone generator with corona cathode discharge
- venturi valve
- ozone turbine mixer
- ozone gas dosage system
- ozone concentration meter.

10. Apparatus according to one of Claims 7 to 9, **characterized in that** both spray devices on the spray booms are equipped on the entire length with UV-C lamps, which generate, in addition to the electromagnetic direct irradiation, hydroxyl compounds in the spray mist which are highly oxidative, have an efficient biocidal effect and enhance the biocidal effect of the ozone treatment according to the hurdle principle.

11. Apparatus according to one of Claims 6 to 10, **characterized in that** all spray booms are equipped with an adaptable spray boom cover such that the spray process can be adjusted, depending on requirements, with respect to the direction (inclination angle) and action of the spray application and the UV-C irradiation.

12. Biocidal use of the method in plant protection according to one of Claims 1 to 5 for protection and destruction of fungi, yeasts, bacteria, viruses, spores, insects and other parasites and their eggs on useful plants.

## Revendications

1. Procédé pour contrôler, dans le cadre de la protection végétale, l'infection par des champignons, des levures, des bactéries, des virus et des insectes, **caractérisé en ce que**, dans une première étape, on mouille des plantes avec un agent mouillant inorganique au moyen d'une technique de pulvérisation électrique dipolaire de jet d'air et on les soumet à un rayonnement de lumière UV-C et, dans une deuxième étape, on les soumet à une projection d'eau contenant de l'ozone au moyen d'une technique de pulvérisation électrique dipolaire de jet d'air et à un rayonnement de lumière UV-C.

2. Procédé selon la revendication 1, **caractérisé en ce que** l'on utilise deux appareils de pulvérisation.

3. Procédé selon la revendication 2, **caractérisé en ce que** l' on humidifie toutes les parties de plante en premier lieu avec de l'eau électriquement chargée et un agent mouillant au moyen d'un premier appareil de pulvérisation.

4. Procédé selon l'une quelconque des revendications 2 ou 3, **caractérisé en ce que** l'on soumet toutes les parties de plante à une pulvérisation avec de l'eau ozonisée dipolaire au moyen d'un deuxième appareil de pulvérisation.

5. Procédé selon l'une quelconque des revendications 1 à 4, **caractérisé en ce que** l'on projette l'eau pulvérisée mouillante à partir d'un premier réservoir et l'eau pulvérisée contenant de l'ozone à partir d'un deuxième réservoir au moyen d'un courant d'air turbulent de jet d'air à partir de tuyères à air spéciales, produit par une turbine à air, une soufflante d'air ou un compresseur d'air, de façon turbulente sur toutes les parties de plante.

6. Dispositif pour la mise en oeuvre du procédé de pulvérisation selon l'une quelconque des revendications 1 à 5, **caractérisé en ce que** le dispositif peut être utilisé selon la technologie de pulvérisation électrique dipolaire et comprend des lampes UV-C ainsi que des tuyères de pulvérisation et des tuyères à courant turbulent de jet d'air pour le mouillage avec un agent mouillant inorganique et comprend des tuyères de pulvérisation avec la technique du courant turbulent de jet d'air pour l'application d'eau ozonisée.

7. Dispositif selon la revendication 6, **caractérisé en ce que** qu'il se compose de deux appareils de pulvérisation.

8. Dispositif selon la revendication 7, **caractérisé en ce qu'**un premier des deux appareils de pulvérisation sert pour le prémouillage des plantes avec de l'eau électriquement chargée négativement avec un agent mouillant et comprend les parties techniques principales suivantes:
- un châssis de montage 3 points avec cadre et support de réservoir
- un réservoir d'eau isolé
- un transformateur électrique
- une anode dans le réservoir d'eau avec câble de raccordement et fusible
- des rampes de pulvérisation télescopiques latérales avec tuyères spéciales à air et à eau, y compris conduites
- des lampes UV-C avec conducteurs électriques
- une pompe de refoulement d'eau avec des soupapes de refoulement et des manomètres, une commande et des robinets, raccordée par des conduites au réservoir d'eau et aux rampes de pulvérisation
- une soufflante d'air ou un compresseur d'air avec une commande, raccordé(e) par des conduites aux tuyères à air sur les rampes de pulvérisation, pour le tourbillonnement du brouillard de pulvérisation
- un arbre d'entraînement ou un entraînement électrique
- des soupapes de refoulement réversibles et de drainage avec robinet
- des couvercles de rampes de pulvérisation, réglables.

9. Dispositif selon la revendication 7, **caractérisé en ce qu'**un deuxième des deux appareils de pulvérisation sert pour l'application d'eau ozonisée et comprend les parties techniques principales suivantes:
- un châssis de montage 3 points avec cadre et support de réservoir, ou un châssis de remorque tracté
- un réservoir d'eau isolé
- des rampes de pulvérisation télescopiques latérales avec tuyères spéciales à air et à eau, y compris conduites ou turbine de pulvérisation d'eau
- des lampes UV-C avec conducteurs électriques
- une pompe de refoulement d'eau avec des soupapes de refoulement et des manomètres, une commande et des robinets, raccordée par des conduites au réservoir d'eau et aux rampes de pulvérisation
- une soufflante d'air ou un compresseur d'air avec une commande, raccordé(e) par des conduites aux tuyères à air sur les rampes de pulvérisation, pour le tourbillonnement du brouillard de pulvérisation
- un arbre d'entraînement ou un entraînement électrique
- des soupapes de refoulement réversibles et de drainage avec robinet
- des couvercles de rampes de pulvérisation, réglables
- un générateur électrique avec armoire de distribution
- une pompe à eau ozonisée
- un séparateur d'air pour la production d'oxygène
- un générateur d'ozone avec une décharge cathodique corona
- une soupape de Venturi
- un mélangeur à turbine pour ozone
- un doseur d'ozone gazeux
- un appareil de mesure de la concentration d'ozone.

10. Dispositif selon l'une quelconque des revendications 7 à 9, **caractérisé en ce que** les deux appareils de pulvérisation sont équipés, sur toute la longueur sur les rampes de pulvérisation, de lampes UV-C qui, en plus du rayonnement direct électromagnétique, produisent dans le brouillard de pulvérisation des composés hydroxyle hautement oxydants, qui ont un effet biocide efficace et renforcent l'action biocide du traitement à l'ozone selon le principe de l'enclos.

11. Dispositif selon l'une quelconque des revendications 6 à 10, **caractérisé en ce que** toutes les rampes de pulvérisation sont munies d'un couvercle de rampe de pulvérisation adaptable, de telle manière que l'opération de pulvérisation, respectivement la direction et l'action de l'application de la pulvérisation et du rayonnement de lumière UV-C puissent être réglées selon les besoins.

12. Application biocide du procédé de protection végétale selon l'une quelconque des revendications 1 à 5 pour la protection et pour la destruction de champignons, levures, bactéries, virus, spores,
insectes et autres nuisibles et de leurs pontes sur des plantes utiles.
